# EUROPEAN PATENT APPLICATION

(11) **EP 1 977 790 A1**
(43) Date of publication of application: **08.10.2008**
(21) Application number: 06843284.8
(22) Date of filing: 26.12.2006
(51) Int. Cl.: A63B 23/18, A61B 5/08, A63B 24/00, A63B 69/00

(54) **RESPIRATION TRAINING MACHINE ENABLING GRASP OF EFFECT**

(30) Priority: 20.01.2006 JP 2006012879
(71) Applicant: Omron Healthcare Co., Ltd., Kyoto 615-0084 (JP); Jichi Medical University, Shimotsuke-shi, Tochigi 329-0498 (JP)
(72) Inventor: SHIRASAKI, Osamu, c/o OMRON HEALTHCARE CO., LTD., Kyoto-shi, Kyoto 615-0084 (JP); SHIGA, Toshikazu, c/o OMRON HEALTHCARE CO., LTD., Kyoto-shi, Kyoto 615-0084 (JP); NAKASE, Yuzo, c/o OMRON HEALTHCARE CO., LTD., Kyoto-shi, Kyoto 615-0084 (JP); KARIO, Kazuomi, c/o JICHI MEDICAL UNIVERSITY, Shimotsuke-shi, Tochigi 329-0498 (JP)
(74) Representative: Wilhelms · Kilian & Partner Patentanwälte
(86) International application number: PCT/JP2006/325900
(87) International publication number: WO 2007/083492

(57) **Abstract**

A breathing exerciser (100) includes: a guide unit (201) for guiding an exercise pattern of breathing to a user; a biological information detecting unit (30) for detecting biological information on the user; a biological information detection control unit (202) for controlling detection of the biological information at timing associated with an exercise period during which the exercise pattern is guided; a characteristic value calculating unit (203) for calculating a characteristic value that reflects an exercise index, based on the biological information detected according to the control of the biological information detection control unit, the exercise index being a target of breathing exercise; an effect index calculating unit (204) for calculating an effect index based on at least two characteristic values, the effect index representing an effect of the breathing exercise; and an informing unit (206) for informing the effect index to the user.

## Description

### TECHNICAL FIELD

The present invention relates to a breathing exerciser and more particularly to a breathing exerciser that guides an exercise pattern of breathing.

### BACKGROUND ART

It has been verified that slow deep breathing leads to suppression of autonomic nerves, providing an effect of decreasing blood pressure. For example, the document "Slow Breathing Improves Arterial Baroreflex Sensitivity and Decreases Blood Pressure in Essential Hypertension" (hereinafter, referred to as the "Non-Patent Document 1 ") by Chacko N.Joseph, et al., in "Hypertension, October 2005", Volume 46, pp. 714-718, published by the American Heart Association, can be a reference document. Therefore, conventionally, as autonomic nervous system exercise methods and biofeedback, studies have been conducted. Also, a number of breathing exercisers for that have been proposed.

Japanese Patent Application Laid-Open No. 62-277976 (hereinafter, referred to as the "Patent Document 1") has disclosed an invention related to an abdominal breathing exercising apparatus in which a sensor that detects movement of the abdominal cavity caused by subject's abdominal breathing is placed on his/her abdomen, a predetermined ideal breathing exercise pattern is generated, an actual breathing pattern is compared with the ideal breathing exercise pattern to determine the degree of matching, and a result of the determination is informed by sound or photoelectric display. Japanese Patent Application Laid-Open No. 2002-301047 (hereinafter, referred to as the "Patent Document 2") has disclosed an invention related to a breathing induction apparatus including a breathing detecting means of detecting breathing of a living body, in which breathing information is extracted from a detected breathing signal, the information is compared for determination with target breathing pattern information to be induced, and a stimulus signal to be provided to the living body is controlled by a correction value which is based on a difference obtained by the comparison. Published Japanese Translation of PCT Application No. 2005-535378 (hereinafter, referred to as the "Patent Document 3") has disclosed that during breathing exercise one or more timing parameters for operation of breathing are changed.

It has become clear that respiratory standstill during sleep due to airway obstruction or autonomic nervous system abnormalities, which is a so-called "Sleep Apnea Syndrome (SAS)", not only simply reduces sleep quality, causing drowsiness during daytime active hours but also promotes hypertension and thereby induces harmful blood pressure fluctuations, which becomes a cause of many serious diseases such as heart diseases and brain diseases.

As treatment approaches for the SAS, there have been proposed an apparatus (CPAP) that delivers a positive pressure of air to the obstructed airway, a surgical operation for expanding the airway, medical treatment (application of an alveolar surfactant preparation to the posterior region of the pharynx (see Published Japanese Translation of PCT Application No. 2001-507364 (hereinafter, referred to as the "Patent Document 4")), enhancement of the muscle groups by muscle strength stimulation exercise by low frequency vibration of the muscle groups of the tongue root in the cervical region (see Japanese Patent Application Laid-Open No. 2005-237807 (hereinafter, referred to as the "Patent Document 5")), etc. However, any of the approaches is a great burden for patients and the current state is that low-burden approaches to the prevention of the SAS have not been proposed.

Therefore, for the prevention of the SAS also, breathing exercise that patients can easily do is considered to be useful.
[Patent Document 1] Japanese Patent Application Laid-Open No. 62-277976
[Patent Document 2] Japanese Patent Application Laid-Open No. 2002-301047
[Patent Document 3] Published Japanese Translation of PCT Application No. 2005-535378
[Patent Document 4] Published Japanese Translation of PCT Application No. 2001-507364
[Patent Document 5] Japanese Patent Application Laid-Open No. 2005-237807
[Non-Patent Document 1] Chacko N.Joseph, Cesare Porta, Gaia Casucci, Nadia Casiraghi, Mara Maffeis, Marco Rossi, Luciano Bernardi, "Slow Breathing Improves Arterial Baroreflex Sensitivity and Decreases Blood Pressure in Essential Hypertension", "Hypertension, October 2005", the American Heart Association, Volume 46, pp. 714-718

### DISCLOSURE OF THE INVENTION

### PROBLEMS TO BE SOLVED BY THE INVENTION

However, the Patent Documents 1 to 3 all have disclosed techniques for merely approximating an actual breathing state to an ideal breathing pattern and thus there is a problem that even if exercise is done with them, a user cannot grasp a final effect (e.g., a decrease in blood pressure, an increase in blood pressure stability, or the like). This is because it is difficult to grasp a cause-and-effect relationship between such an exercise action as breathing and an improvement target (e.g., a blood pressure value). Hence, there is a concern that it may be difficult to maintain exercise continuity. Furthermore, since the user needs to use a sensor that detects breathing, there is a problem that handling thereof is troublesome.

The present invention is made to solve problems such as those described above and an object of the present invention is therefore to provide a breathing exerciser that enables a user to easily grasp an effect brought about by breathing exercise.

### MEANS FOR SOLVING THE PROBLEMS

A breathing exerciser according to one aspect of the present invention comprises: a guide unit for guiding an exercise pattern of breathing to a user; a detecting unit for detecting biological information on the user; a detection control unit for controlling detection of the biological information at timing associated with an exercise period during which the exercise pattern is guided; a characteristic value calculating unit for calculating a characteristic value that reflects an exercise index, based on the biological information detected according to the control of the detection control unit, the exercise index being a target of breathing exercise; an effect index calculating unit for calculating an effect index based on at least two characteristic values, the effect index representing an effect of the breathing exercise; and an informing unit for informing the effect index to the user.

Preferably, the detection control unit allows the detecting unit to detect biological information before and after the exercise period, the characteristic value calculating unit calculates two characteristic values based on the detected biological information respectively for before and after the exercise period, and the effect index calculating unit calculates an effect index for before and after the exercise period, based on the calculated characteristic values.

Preferably, the breathing exerciser further comprises: a storage unit for storing a characteristic value which is based on biological information detected before or after a previous exercise period, the detection control unit allows the detecting unit to detect biological information before or after the exercise period, the characteristic value calculating unit calculates two characteristic values based on the detected biological information and the biological information stored in the storage unit, and the effect index calculating unit calculates an effect index for before each exercise period or for after each exercise period, based on the calculated characteristic values.

Preferably, the detection control unit allows the detecting unit to detect biological information during the exercise period, the characteristic value calculating unit calculates a characteristic value based on the detected biological information for during the exercise period, and the effect index calculating unit calculates an effect index for during the exercise period, based on the calculated characteristic value.

Preferably, the breathing exerciser further comprises: a changing unit for changing the exercise pattern based on a result of comparison between the effect index for during the exercise period which is calculated by the effect index calculating unit and a predetermined threshold.

Preferably, the exercise index and the characteristic value each are a blood pressure value, and the effect index is a degree of decrease in the blood pressure value.

Preferably, the exercise index is an autonomic nervous activity level index representing an autonomic nervous activity level, the characteristic value includes a heart rate fluctuation, the characteristic value calculating unit calculates heart rate fluctuations based on two heart rates respectively corresponding to two pieces of detected biological information, and the effect index calculating unit calculates, as the effect index, a degree of suppression of the autonomic nervous activity level based on the two heart rate fluctuations.

Preferably, the exercise index is a blood pressure stability index, the characteristic value includes a baroreflex sensitivity, the characteristic value calculating unit calculates baroreflex sensitivities based on a heart rate fluctuation and a blood pressure fluctuation respectively corresponding to two pieces of detected biological information, and the effect index calculating unit calculates, as the effect index, a degree of increase in blood pressure stability based on a difference or ratio between the two baroreflex sensitivities.

Preferably, the breathing exerciser further comprises: a storage unit for storing an effect index associated with a previous exercise period, and the informing unit further informs a trend between the stored effect index and the calculated effect index.

A breathing exerciser according to another aspect of the present invention comprises: a guide unit for guiding an exercise pattern of breathing to a user; a detecting unit for detecting biological information on the user; a detection control unit for controlling detection of the biological information at timing associated with an exercise period during which the exercise pattern is guided; a characteristic value calculating unit for calculating a characteristic value that reflects an exercise index, based on the biological information detected according to the control of the detection control unit, the exercise index being a target of breathing exercise; and an informing unit for informing at least two characteristic values to the user.

Preferably, the detection control unit allows the detecting unit to detect biological information before and after the exercise period, the characteristic value calculating unit calculates two characteristic values based on the detected biological information respectively for before and after the exercise period, and the informing unit informs the characteristic values respectively for before and after the exercise period.

Preferably, the breathing exerciser further comprises: a storage unit for storing a characteristic value which is based on biological information detected before or after a previous exercise period, and the detection control unit allows the detecting unit to detect biological information before or after the exercise period, the characteristic value calculating unit calculates two characteristic values based on the detected biological information and the biological information stored in the storage unit, and the informing unit informs the characteristic values for before each exercise period or for after each exercise period.

Preferably, the detection control unit allows the detecting unit to detect biological information during the exercise period, the characteristic value calculating unit calculates a characteristic value based on the detected biological information for during the exercise period, and the informing unit informs the characteristic value for during the exercise period.

Preferably, the breathing exerciser further comprises: a changing unit for changing the exercise pattern based on a result of comparison between the characteristic value for during the exercise period which is calculated by the characteristic value calculating unit and a predetermined threshold.

Preferably, the exercise index and the characteristic value each are a blood pressure value.
Preferably, the exercise index is an autonomic nervous activity level index representing an autonomic nervous activity level, the characteristic value includes a heart rate fluctuation, and the characteristic value calculating unit calculates heart rate fluctuations based on two heart rates respectively corresponding to two pieces of detected biological information.

Preferably, the exercise index is a blood pressure stability index, the characteristic value includes a baroreflex sensitivity, and the characteristic value calculating unit calculates baroreflex sensitivities based on a heart rate fluctuation and a blood pressure fluctuation respectively corresponding to two pieces of detected biological information.

Preferably, the breathing exerciser further comprises: an input unit for inputting physical information on the user; and a determining unit for determining an exercise pattern based on the inputted physical information, wherein the guide unit guides the determined exercise pattern.

Preferably, the determining unit determines the exercise pattern based on an effect index associated with a previous exercise period.

### EFFECT OF THE INVENTION

According to the present invention, the user can grasp an effect brought about by breathing exercise.

### BRIEF DESCRIPTION OF THE DRAWINGS

FIG. 1 is a diagram showing a schematic view of a breathing exerciser in first and second embodiments of the present invention.
FIG. 2 is a block diagram showing a configuration of the breathing exerciser in the first and second embodiments of the present invention.
FIG. 3A is a diagram showing an exemplary structure of an execution pattern information storage unit in the first and second embodiments of the present invention.
FIG. 3B is a diagram showing an exemplary structure of an exercise result storage unit in the first and second embodiments of the present invention.
FIG. 4 is a flowchart showing a flow of a breathing exercise process in the first embodiment of the present invention.
FIG. 5 is a flowchart showing an exercise pattern determination process in the first embodiment of the present invention.
FIG. 6 is a conceptual diagram showing load levels of eight exercise patterns stored in an execution pattern information storage unit 124.
FIG. 7 is a diagram for specifically describing exercise patterns.
FIG. 8 is a diagram showing an example of a screen to be displayed when inputting physical information.
FIG. 9 is a diagram showing an exemplary display of a breathing guide.
FIG. 10 is a diagram showing a detailed exemplary display of the breathing guide based on an exercise pattern for during an exercise period and a diagram showing an example of the exercise pattern.
FIG. 11 is a diagram showing an exemplary display of information on an effect index for before exercise or after exercise.
FIG. 12 is a diagram showing an exemplary display of information on an effect index for before and after exercise.
FIG. 13 is a diagram showing an exemplary display of a trend of an effect index.
FIG. 14 is a flowchart showing a flow of a breathing exercise process in the second embodiment of the present invention.
FIG. 15 is a diagram showing a pattern change process in the second embodiment of the present invention.

### DESCRIPTION OF REFERENCE NUMERALS

1: exerciser main body, 2: cuff, 3: air tube, 4: display unit, 12: memory, 13: timer, 14: pressure sensor, 15: oscillator circuit, 16: pump, 17: pump drive circuit, 18: valve, 19: valve drive circuit, 20: control unit, 21: operation unit, 25: air bag, 24: audio output unit, 30: biological information detecting unit, 100: breathing exerciser, 122: pattern storage unit, 124: execution pattern information storage unit, 126: exercise result storage unit, 201: guide unit, 202: biological information detection control unit, 203: characteristic value calculating unit, 204: effect index calculating unit, 206: informing unit, and 208: pattern changing unit.

### BEST MODE FOR CARRYING OUT THE INVENTION

Embodiments of the present invention will be described in detail with reference to the drawings. Note that the same or corresponding parts are denoted by the same reference numerals throughout the drawings.

[First Embodiment]

### (For configuration)

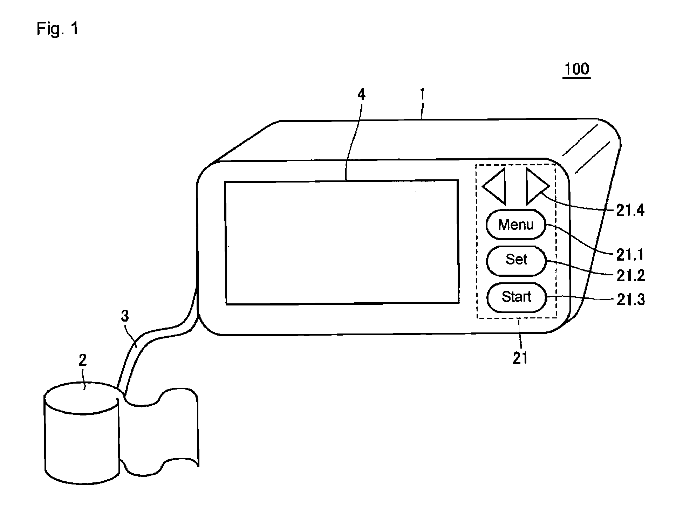

FIG. 1 is a diagram showing a schematic view of a breathing exerciser 100 in a first embodiment of the present invention.

Referring to FIG. 1, the breathing exerciser 100 according to the present embodiment includes an exerciser main body 1; a cuff 2 which is placed on a predetermined body part of a user to pressurize by air pressure; and an air tube 3 connecting the exerciser main body 1 and the cuff 2.

The exerciser main body 1 includes a display unit 4 provided so that the user can check display content; and an operation unit 21 provided so that the user can externally operate the exerciser. The operation unit 21 includes a plurality of switches and has a menu switch 21.1 for inputting an instruction to display a variety of menus of the breathing exerciser 100; a set switch 21.2 for inputting an instruction to perform a menu or each operation; a start switch 21.3 for inputting an instruction to start exercise; left and right scroll switches 21.4; and the like.

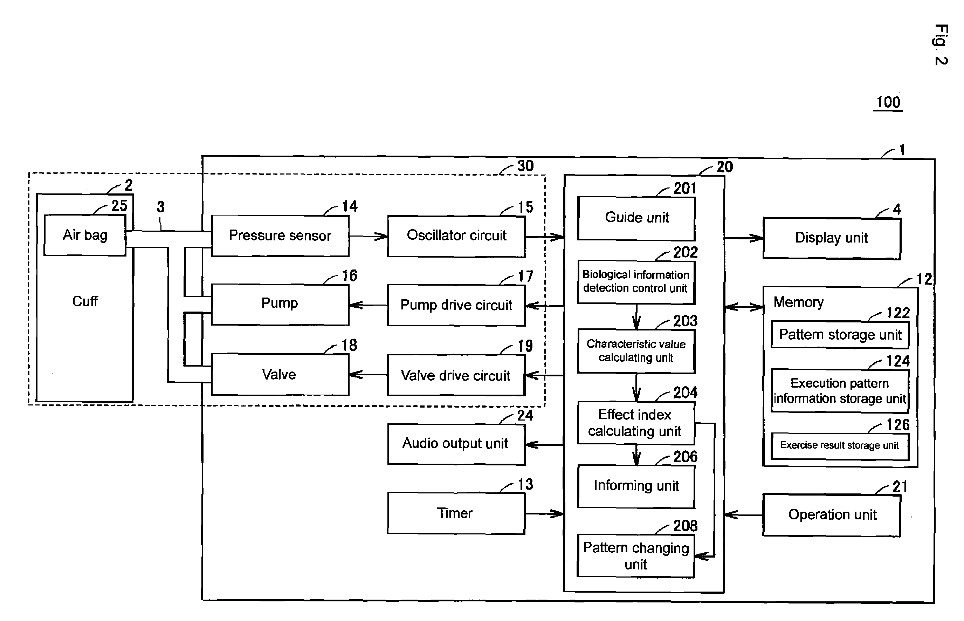

FIG. 2 is a block diagram showing a configuration of the breathing exerciser 100 in the first embodiment of the present invention. Referring to FIG. 2, the breathing exerciser 100 includes a biological information detecting unit 30 for detecting biological information on the user; a control unit 20 for intensively controlling and monitoring each unit; a display unit 4; a memory 12 in which various data and programs are to be stored; an operation unit 21; a timer 13 that performs a timer operation to output timer data; and an audio output unit 24. In the present embodiment, the biological information detecting unit 30 includes a cuff 2; an air bag 25 contained in the cuff 2; a pressure sensor 14, the capacity of which changes by pressure (hereinafter, referred to as "cuff pressure") in the air bag 25; an oscillator circuit 15 that outputs a signal with an oscillation frequency depending on a capacitance value of the pressure sensor 14, to the control unit 20; a pump 16 and a valve 18 for adjusting the level of the cuff pressure; a pump drive circuit 17 that drives the pump 16; and a valve drive circuit 19 for adjusting the degree of open/close of the valve 18. The air bag 25 is connected to the pressure sensor 14, the pump 16, and the valve 18 via an air tube 3. Note that the biological information detecting unit 30 is not limited to such a configuration and any configuration can be employed as long as biological information for calculating a characteristic value, which will be described later, can be detected.

The control unit 20 is configured by, for example, a CPU (Central Processing Unit). The control unit 20 includes a guide unit 201 for guiding an exercise pattern of breathing to the user; a biological information detection control unit 202 for controlling detection of biological information at timing associated with an exercise period; a characteristic value calculating unit 203 for calculating a biological characteristic value that reflects an exercise index, based on the detected biological information; an effect index calculating unit 204 for calculating an effect index based on at least two characteristic values calculated by the characteristic value calculating unit 203; and an informing unit 206 for informing the effect index calculated by the effect index calculating unit 204 to the user.

Here, the "exercise index" is an index serving as a target (improvement target) for breathing exercise and represents, for example, a blood pressure value, an autonomic nervous activity level index, a blood pressure stability index, etc. The "effect index" is an index representing an effect of breathing exercise. The effect index for when the exercise index is a blood pressure value is the degree of decrease in blood pressure value. The effect index for when the exercise index is an autonomic nervous activity level index is the degree of suppression of an autonomic nervous activity level. The effect index for when the exercise index is a blood pressure stability index is the degree of increase in blood pressure stability. In the present embodiment, description is made with the exercise index being a blood pressure value.

The "exercise period" is a period during which an exercise pattern is guided by the guide unit 201.

Note that a pattern changing unit 208 shown in FIG. 2 is shown to describe a second embodiment, which will be described later, and thus does not need to be included in the control unit 20 of the breathing exerciser 100 in the first embodiment.

The guide unit 201 specifically performs a process of displaying information (hereinafter, also referred to as a "breathing guide") for guiding to breathing which is based on an exercise pattern, on the display unit 4. Note that although in the present embodiment description is made such that an exercise pattern is guided using the display unit 4, an exercise pattern may be guided by sound by the audio output unit 24.

The biological information detection control unit 202 performs detection control of biological information, i.e., pulse wave information, at timing associated with an exercise period. Specifically, the biological information detection control unit 202 controls the drive of the pump drive circuit 17 and the valve drive circuit 19 to convert a signal obtained from the oscillator circuit 15 into a pressure signal and thereby detects pressure.

The characteristic value calculating unit 203 specifically calculates a blood pressure value (systolic blood pressure and diastolic blood pressure) by applying a predetermined algorithm to detected pressure data (pulse wave information). As such, as a characteristic value for when the exercise index is a blood pressure value, a blood pressure value which is the same as the exercise index is calculated. For a procedure of calculation of a blood pressure value, any known procedure which is conventionally provided can be used. Note that with the above-described configuration pulse rate (heart rate) can also be calculated by applying a predetermined algorithm to detected pressure data.

The effect index calculating unit 204 calculates, based on calculated characteristic values, effect indices respectively for before exercise, during exercise, after exercise, and before and after exercise. Each effect index is specifically calculated using, for example, a difference between two characteristic values or a ratio between two characteristic values. In the present embodiment, as an effect index, the degree of decrease in blood pressure value (ΔBP) is calculated.

An effect index for before exercise is calculated using a characteristic value which is based on biological information detected before (immediately before) a previous exercise period and a characteristic value which is based on biological information detected before (immediately before) a current exercise period. The "previous exercise period" refers to an exercise period in a breathing exercise process which is performed earlier than a current breathing exercise process, and may be, for example, the first exercise period or may be the last exercise period. In the following description, it is assumed that an effect index for before each exercise is calculated based on a characteristic value obtained before the first exercise period and a characteristic value obtained before a current exercise period.

An effect index for during exercise is calculated using characteristic values which are based on biological information detected during an exercise period by the biological information detection control unit 202. For example, an effect index for during exercise is calculated based on two characteristic values obtained during an exercise period. Alternatively, an effect index for during exercise may be calculated based on a characteristic value obtained before an exercise period and a characteristic value obtained during the exercise period.

An effect index for after each exercise is, as with the effect index for before exercise, calculated using a characteristic value which is based on biological information detected after (immediately after) a previous exercise period and a characteristic value which is based on biological information detected after (immediately after) a current exercise period. In the following description, it is assumed that an effect index for after each exercise is calculated based on a characteristic value obtained after the first exercise period and a characteristic value obtained after a current exercise period.

An effect index for before and after exercise is calculated using a characteristic value which is based on biological information detected before a current exercise period and a characteristic value which is based on biological information detected after the current exercise period.

Although in the present embodiment effect indices are thus calculated before exercise, during an exercise period, after exercise, and before and after exercise, at least one of such effect indices may be calculated. Alternatively, in addition to them/instead of them, for example, an effect index may be calculated based on a characteristic value obtained during a current exercise period and a characteristic value obtained after the current exercise period.

The control unit 20 may further calculate a change (trend) of the effect of breathing exercise, based on a difference between a past effect index and a current effect index. Alternatively, the control unit 20 may compute a current target value based on a predetermined computational expression and a past effect index to calculate a shift of a current effect index from the target value.

The informing unit 206 specifically performs a process of displaying information on a calculated effect index, on the display unit 4. The informing unit 206 may further display a trend of the effect index on the display unit 4.

The operation of each block in the control unit 20 may be implemented by executing software stored in the memory 12 or at least one of the blocks may be implemented by hardware.

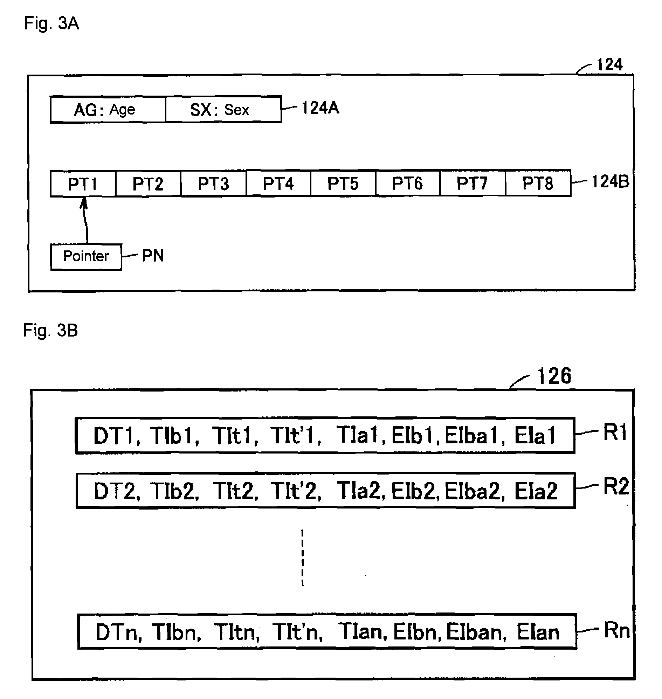

The memory 12 is a non-volatile memory, e.g., flash memory. The memory 12 includes a pattern storage unit 122 in which data on a plurality of exercise patterns are stored in advance; an execution pattern information storage unit 124 for storing information about an exercise pattern (hereinafter, also referred to as an "execution pattern") to be executed in user's breathing exercise; and an exercise result storage unit 126 for storing exercise results. Note that the storage units do not need to be included in the same storage medium (memory 12) and may be included in different storage media. Specific examples of data structures in the execution pattern information storage unit 124 and the exercise result storage unit 126 will be described with reference to FIGS. 3A and 3B.

FIGS. 3A and 3B are diagrams respectively showing exemplary structures of the execution pattern information storage unit 124 and the exercise result storage unit 126 in the first embodiment of the present invention.

Referring to FIG. 3A, the execution pattern information storage unit 124 includes a storage area 124A for storing user's physical information; and a storage area 124B for storing data on exercise patterns to be executed. In the storage area 124A, user's age data AG and sex data SX are stored. In the storage area 124B, for example, data PT1 to PT8 on eight exercise patterns is stored in ascending order of load levels. The data on eight exercise patterns is selected from data on a plurality of exercise patterns stored in the pattern storage unit 122. Note that a specific selection method will be described later.

The exercise pattern data includes information on at least exercise time or the number of breaths, a breathing cycle, and the depth of breathing. The load level is determined by, for example, at least one of parameters including at least exercise time or the number of breaths, a breathing cycle, and the depth of breathing. In the present embodiment, it is assumed that the load level is determined by a breathing cycle (the number of breaths per certain period of time).

The execution pattern information storage unit 124 further includes a pointer PN that points to data on an exercise pattern to be executed this time (or next time) among the data PT1 to PT8 on eight exercise patterns stored in the storage area 124B. Note that although here management of an exercise pattern to be executed is performed by the pointer PN, the present invention is not limited to such a technique as long as which exercise pattern is executed this time or which exercise pattern has been executed last time can be identified.

Referring to FIG. 3B, in the exercise result storage unit 126, exercise results are stored in the unit of record R. The record R includes date and time data DT indicating exercise data and time; blood pressure data Tlb indicating a blood pressure value (characteristic value) for before exercise; blood pressure data Tlt and Tlt' indicating blood pressure values (characteristics values) for during exercise; blood pressure data Tla indicating a blood pressure value (characteristic value) for after exercise; effect index data Elb indicating the degree of decrease in blood pressure value (effect index) for before exercise; effect index data Elba indicating the degree of decrease in blood pressure value (effect index) for before and after exercise; and effect index data Ela indicating the degree of decrease in blood pressure value (effect index) for after each exercise. Note that such data is not limited to a storage format using the record R as long as the data is stored so as to be associated with each exercise.

(For operation)

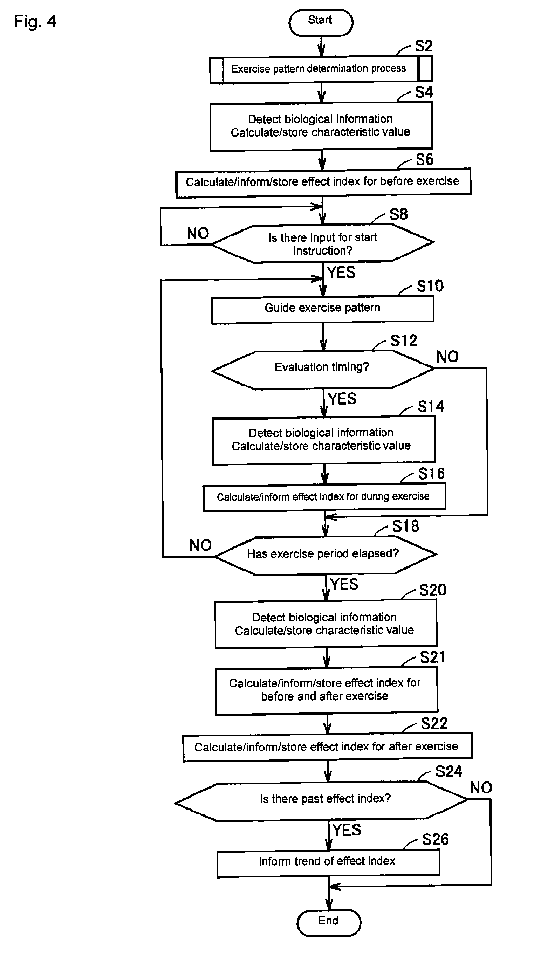

FIG. 4 is a flowchart showing a flow of a breathing exercise process in the first embodiment of the present invention. The process shown in the flowchart in FIG. 4 is stored in advance in the memory 12 as a program and a function of the breathing exercise process is implemented by the control unit 20 reading and executing this program. Note that for simplification of description it is assumed that a series of breathing exercise process for this time is an nth one (n: natural number).

Referring to FIG. 4, a flow of a processing operation will be described below. First, an exercise pattern determination process which will be described later using a subroutine is performed (step S2).

Then, a biological information detection process and a characteristic value calculation/storage process are performed (step S4). Specifically, first, the biological information detection control unit 202 performs control to detect biological information and the characteristic value calculating unit 203 calculates a characteristic value, i.e., a blood pressure value (e.g., a systolic blood pressure value), based on detected biological information (pulse wave information). Then, data on the calculated blood pressure value (data on the characteristic value) is stored in the exercise result storage unit 126 as blood pressure data Tlbn. Note that at the time of or before detecting biological information, the user may be urged to take a deep breath.

Subsequently, a calculation/informing/storage process of an effect index for before each exercise is performed (step S6). Specifically, the effect index calculating unit 204 calculates an effect index, i.e., the degree of decrease in blood pressure value, for before each exercise, based on blood pressure data Tlbn for this time which is calculated and stored in step S4 and blood pressure data Tlb1 for the first time. Information indicating the calculated degree of decrease in blood pressure value is displayed in a predetermined area of the display unit 4. By this, the degree of decrease in blood pressure value for this time with reference to a blood pressure value for the first time can be informed to the user. Furthermore, the calculated degree of decrease in blood pressure value for before each exercise is stored as effect index data Elbn.

Next, it is determined whether an instruction to start exercise has been inputted (step S8). Specifically, it is determined whether the start switch 21.3 has been pressed by the user. The control unit 20 waits until an input of a start instruction has been detected (NO in step S8). If an input of a start instruction has been detected (YES in step S8), then processing proceeds to step S10. Note that although here breathing exercise starts after an instruction from the user has been inputted, breathing exercise may automatically start after an effect index has been informed (after the process in step S6).

In step S10, the guide unit 201 guides an exercise pattern determined in step S2 to the user. Specifically, a breathing guide (e.g., how much more exhalation or inhalation should be performed) is displayed on the display unit 4 based on exercise pattern data pointed to by the pointer PN in the execution pattern information storage unit 124.

Then, it is determined whether evaluation timing has come (step S12). The evaluation timing may be predetermined such as every five minutes, for example, after the start of exercise, or whether it is evaluation timing may be determined by an instruction from the user. If it is determined that evaluation timing has come (YES in step S12), then processing proceeds to step S14. On the other hand, if it is determined that evaluation time has not come (NO in step S12), then processing proceeds to step S18.

In step S14, a biological information detection process and a characteristic value calculation/storage process are performed again. The processes for biological information detection and characteristic value calculation here can be performed in the same manner as that for step S4. A calculated characteristic value (data on a blood pressure value) is stored in the exercise result storage unit 126 as blood pressure data Tltn (blood pressure data Tlt'n if it is a second time).

Then, a calculation/informing process of an effect index for during exercise is performed (step S16). In step S16, specifically, the effect index calculating unit 204 calculates an effect index, i.e., the degree of decrease in blood pressure value, for during exercise based on, for example, the blood pressure data Tltn and blood pressure data Tlt'n calculated and stored in step S14. Alternatively, an effect index for during exercise may be calculated based on the blood pressure data Tlbn calculated and stored in step S4 and the blood pressure data Tltn calculated and stored in step S14. Information on the calculated degree of decrease in blood pressure value is displayed in a predetermined area of the display unit 4. By this, the degree of decrease in blood pressure value for during exercise can be informed to the user.

In step S18, it is determined whether an exercise period has elapsed. If an exercise period has not elapsed (NO in step S18), then processing returns to step S10. On the other hand, if it is determined that an exercise period has elapsed (YES in step S18), then processing proceeds to step S20.

In step S20, a biological information detection process and a characteristic value calculation/storage process are further performed. The processes for biological information detection and characteristic value calculation here can also be performed in the same manner as that for step S4. A calculated characteristic value (data on a blood pressure value) is stored in the exercise result storage unit 126 as blood pressure data Tlan. Note that at the time of or before detecting biological information in step S20, the user may be urged to take a deep breath.

Subsequently, a calculation/informing/storage process of an effect index for before and after exercise is performed (step S21). Specifically, the effect index calculating unit 204 calculates an effect index, i.e., the degree of decrease in blood pressure value, for before and after exercise based on the blood pressure data Tlbn for before exercise which is calculated and stored in step S4 and the blood pressure data Tlan for after exercise which is calculated and stored in step S20. Information on the calculated degree of decrease in blood pressure value is displayed in a predetermined area of the display unit 4. By this, the degree of decrease in blood pressure value for after exercise with reference to a blood pressure value for before exercise can be informed to the user. Furthermore, the calculated degree of decrease in blood pressure value for before and after exercise is stored as effect index data Elban.

Furthermore, a calculation/informing/storage process of an effect index for after each exercise is performed (step S22). Specifically, the effect index calculating unit 204 calculates an effect index, i.e., the degree of decrease in blood pressure value, for after each exercise based on the blood pressure data Tlan for this time which is calculated and stored in step S20 and the blood pressure data Tla1 for the first time. Information on the calculated degree of decrease in blood pressure value is displayed in a predetermined area of the display unit 4. By this, the degree of decrease in blood pressure value for this time with reference to the blood pressure value for the first time can be informed to the user. Furthermore, the calculated degree of decrease in blood pressure value for after each exercise is stored as effect index data Elan.

Next, it is determined whether there is a past effect index (an effect index associated with a previous exercise period) (step S24). If it is determined that a past effect index is stored in the exercise result storage unit 126 (YES in step S24), then processing proceeds to step S26. On the other hand, if a past effect index is not stored (NO in step S24), then the breathing exercise process ends.

In step S26, the informing unit 206 displays a trend between the past effect index and the effect index for this time on the display unit 4. By this, the trend of the effect index is informed to the user. When the process in step S26 is completed, the series of breathing exercise process ends.

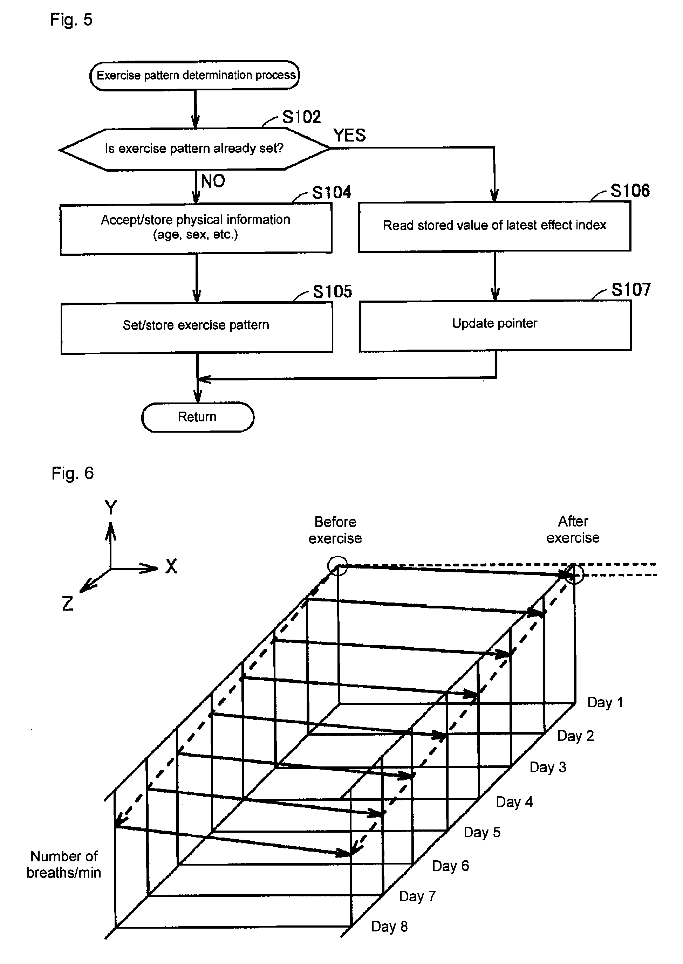

Next, the exercise pattern determination process (S2) will be described. FIG. 5 is a flowchart showing the exercise pattern determination process in the first embodiment of the present invention.

Referring to FIG. 5, first, the control unit 20 determines whether an exercise pattern is already set (step S102). If an exercise pattern is not already set (NO in step S102), then processing proceeds to step S104. On the other hand, if an exercise pattern is already set (NO in step S102), then processing proceeds to step S106.

In step S104, the control unit 20 accepts an input of physical information (e.g., age, sex, etc.) from the user and stores the physical information in the storage area 124A of the execution pattern information storage unit 124. When the process in step S104 is completed, processing proceeds to step S105.

In step S105, an exercise pattern setting/storage process is performed based on the accepted physical information. Specifically, for example, a process such as that below is performed. An association table in which age and sex are associated with identification information on an exercise pattern is stored in advance in, for example, the pattern storage unit 122. The control unit 20 identifies identification information associated with user's age and sex in the association table. Then, data on an exercise pattern indicated by the identified identification information is read from the pattern storage unit 122 and stored in the storage area 124B of the execution pattern information storage unit 124. Note that in the association table eight pieces of identification information respectively indicating eight exercise patterns may be stored so as to be associated with each physical information or identification information indicating one exercise pattern may be stored so as to be associated with each of physical information. In the latter case, for example, data on an exercise pattern indicated by identified identification information and data on seven exercise patterns with load levels continuous with a load level of the exercise pattern may be selected. In such a case, the pointer PN is set so as to point to data PT1 on an exercise pattern.

In FIG. 6, an x-axis shows the exercise time per exercise, a y-axis shows the load level (the number of breaths per certain period of time (e.g., minute) (i.e., a breathing cycle)), and a z-axis shows the number of days (the number of exercises). As shown in FIG. 6, exercise patterns are set such that the load level increases as the number of days (the number of exercises) increases, i.e., such that the number of breathes per certain period of time decreases. Exercise pattern data PT1 to PT8 respectively correspond to days 1 to 8. Note that in the drawing each exercise pattern is a pattern that the load gradually increases from the start of exercise (the number of breaths per certain period of time decreases). By this, the user can warm up and then do substantial breathing exercise. Note that during an exercise period, the load may be constant. Alternatively, during an exercise period, not only a warm-up period but also a cool-down period may be provided.

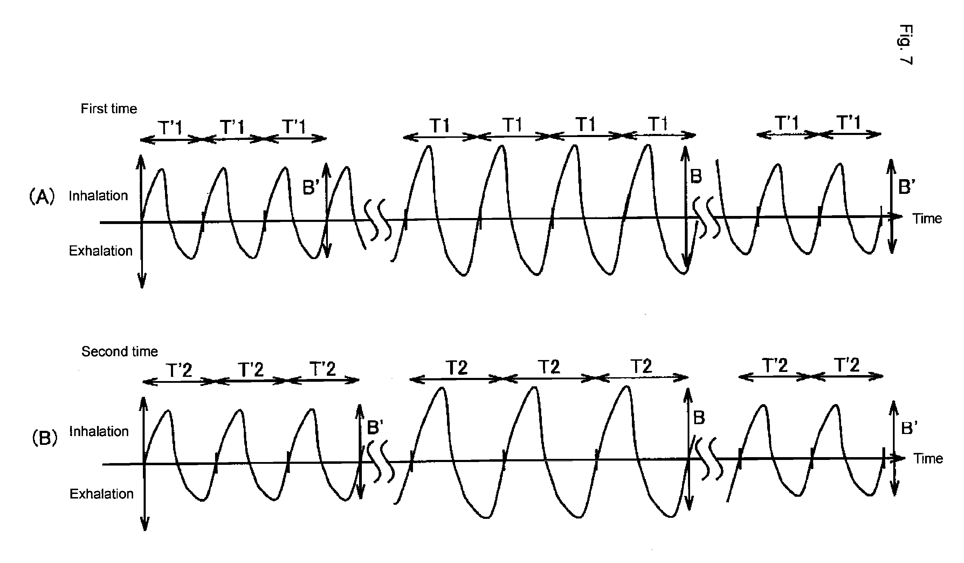

(A) of FIG. 7 shows an example of an exercise pattern for a first time (corresponding to the exercise pattern data PT1), and (B) of FIG. 7 shows an example of an exercise pattern for a second time (corresponding to the exercise pattern data PT2). Note that here an example of the case is shown in which an exercise period includes a warm-up period, a substantial exercise period, and a cool-down period.

Referring to (A) of FIG. 7, a breathing cycle t1 during the substantial exercise period is longer than a breathing cycle t'1 during the warm-up period and the cool-down period. Also, a depth b1 of breathing during the substantial exercise period is greater than a depth b'1 of breathing during the warm-up period and the cool-down period. Also, in (B) of FIG. 7, similarly, a breathing cycle t2 during the substantial exercise period is longer than a breathing cycle t'2 during the warm-up period and the cool-down period. Also, a depth b2 of breathing during the substantial exercise period is greater than a depth b'2 of breathing during the warm-up period and the cool-down period.

Referring to FIG. 7, the breathing cycle t2 during the substantial exercise period in the exercise pattern for the second time is longer than the breathing cycle t1 during the substantial exercise period in the exercise pattern for the first time. As such, the breathing cycle becomes longer as the load level increases.

In step S106, the control unit 20 reads a stored value of the latest effect index. The effect index to be read here is, for example, effect index data Elba (n-1) which indicates the degree of decrease in blood pressure for before and after exercise. When the process in step S106 is completed, processing proceeds to step S107.

In step S107, an update of the pointer PN is performed based on the read effect index data Elba (n-1). When the latest effect index data Elba (n-1) is within a predetermined range, the pointer PN is updated to point to data on a next exercise pattern (an exercise pattern with a one-step higher load level). For example, when the pointer PN points to the exercise pattern data PT2 first, an update is performed to point to the exercise pattern data PT3. On the other hand, when the latest effect index data Elba (n-1) exceeds the predetermined range, for example, a process such as that shown below is performed. Specifically, when the latest effect index data Elba (n-1) exceeds an upper limit of the range, the pointer PN is updated to point to data on an exercise pattern which is the one after the next one (an exercise pattern with a two-step higher load level). For example, when the pointer PN points to the exercise pattern data PT2 first, an update is performed to point to the exercise pattern data PT4. In contrast, when the latest effect index data Elba (n-1) falls below a lower limit of the range, the pointer PN is not updated and is allowed to point to the same exercise pattern data as the last one.

When the process in either step S105 or step S107 is completed, processing is returned to the main routine.

In this manner, an exercise pattern is selected according to the degree of achievement (the degree of improvement/deterioration) of the user him/herself. By this, when the effect index is a good result, the user can also reach a target value earlier. Note that although in the present embodiment when the effect of exercise is high, the load of exercise is further advanced to reach the target value early, the present invention is not limited to such a technique. For example, when the effect is high, in contrast, the user may be allowed to continue exercise with a lower load. Specifically, when the latest effect index data Elba (n-1) exceeds the upper limit, the pointer PN may not be updated and may be allowed to point to the same exercise pattern data as the last one.

Note that although in step S106 a stored value of the latest effect index is read and an exercise pattern is selected based on the read value, an exercise pattern may be selected based on a stored value of the latest characteristic value. In this case, it is desirable to select an exercise pattern based on blood pressure data TIb (n-1) for before exercise that reflects a blood pressure value of the user during the course of a usual day of activities.

Note that, as described above, when the effect index exceeds a predetermined upper limit, an exercise pattern with a two-step higher load level is selected, and when the effect index falls below a lower limit, the same exercise pattern as the last one is selected. However, the present invention is not limited to such a process. For example, when the effect index exceeds a predetermined upper limit, an exercise pattern with a three-step higher load level may be selected, and when the effect index falls below a predetermined threshold which is lower than the above-described upper limit, an exercise pattern may be re-set again.

The upper and lower limits may be determined in advance for each exercise pattern.
When the pointer PN points to the exercise pattern data PT8, and an immediately preceding effect index does not fall below the lower limit, an exercise pattern may be newly re-set. For example, in such a case, exercise pattern data with a one- or more-step higher load level than that of the exercise pattern data PT8 is read from the pattern storage unit 122. The read exercise pattern data may be overwritten in the storage area 124B or may be stored in another storage area. When an exercise pattern is thus newly re-set, physical information stored in the storage area 124A may be used again.

As described above, in the present embodiment, description is made such that data on a plurality of exercise patterns is stored in advance in the pattern storage unit 122. However, the control unit 20 may calculate each parameter of an exercise pattern based on physical information on a subject and a predetermined computational expression. Alternatively, only one exercise pattern may be stored in advance.

Although in the present embodiment setting of a plurality of exercise patterns is performed first, each time exercise is done one exercise pattern may be selected from the pattern storage unit 122.

(For exemplary display)

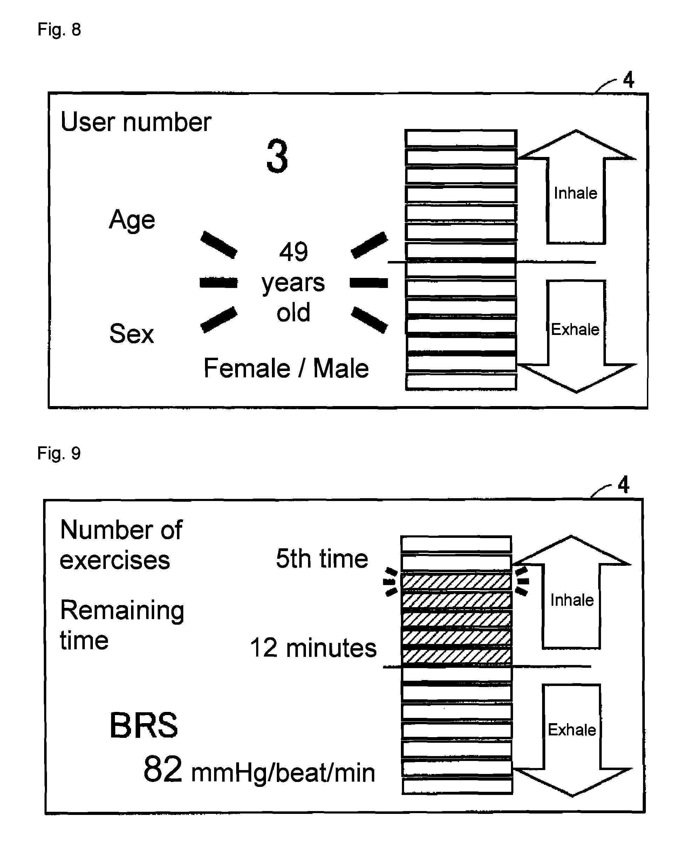

FIG. 8 is a diagram showing an example of a screen to be displayed when inputting physical information in step S104. As shown in FIG. 8, an item (age or sex) of physical information being inputted is displayed blinking. Physical information can be inputted using the scroll switches 21.4 and the set switch 21.2. Note that in order that a plurality of users can use the exerciser a user number may be inputted. In this case, inputted physical information and user number are stored so as to be associated with each other. Similarly, exercise results, etc., are also stored so as to be associated with the user number.

FIG. 9 is a diagram showing an exemplary display of a breathing guide in step S10. Referring to FIG. 9, a breathing guide is performed by highlighting/not highlighting 14 blocks displayed in a vertical direction on the screen. Also, the number of exercises (nth time) and remaining time are displayed in their respective predetermined areas. Note that a characteristic value or an effect index for during exercise may be further displayed. Here, an example is shown in which a characteristic value (BRS which will be described later) is displayed.

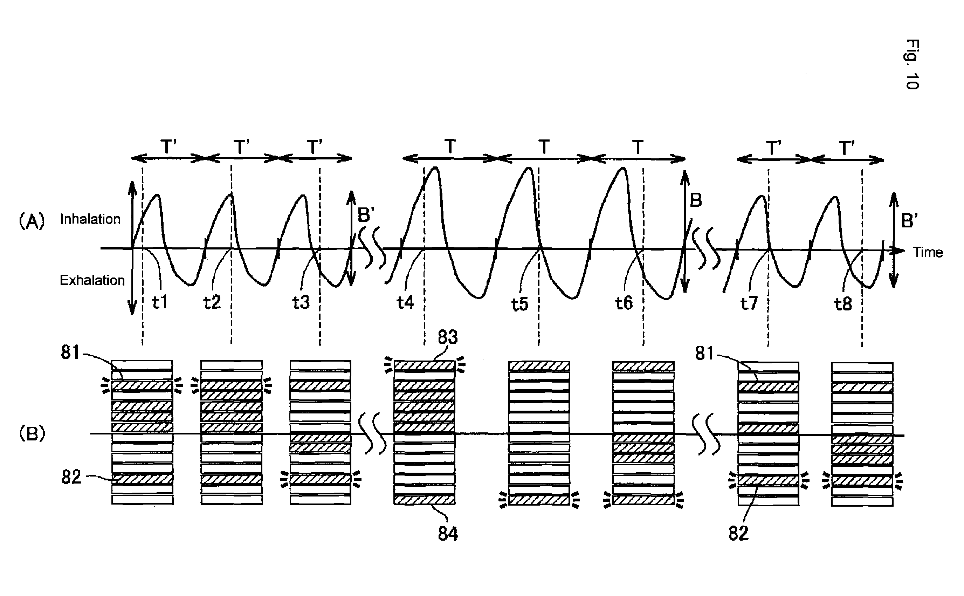

Detailed exemplary display of the breathing guide will be described with reference to FIG. 10. (A) of FIG. 10 is a diagram showing an example of an exercise pattern and (B) is a diagram showing an exemplary display of the breathing guide at arbitrary times t1 to t8 of the exercise pattern shown in (A).

Referring to (B) of FIG. 10, in each breathing guide, a block at a location indicating the depth of breathing is fixedly displayed highlighted (displayed darkened). In the guides at times t1, t2, and t3 during a warm-up period, fifth blocks 81 and 82 respectively located upward and downward from the center are fixedly displayed highlighted. In the breathing guide at time t1, the first to third blocks located upward from the center are displayed highlighted and the block 81 is displayed blinking. By this, it is possible to inform the user of how much more time he/she should inhale. In the breathing guide at time t2, similarly, the block 81 is displayed blinking and all of the first to fourth blocks located upward from the center are displayed highlighted. By this, it is possible to inform that it is the end of an inhalation period.

At times t4, t5, and t6 during a substantial exercise period, seventh blocks (blocks at both ends) 83 and 84 respectively located upward and downward from the center are fixedly displayed highlighted. In the breathing guide at time t5, the block 84 is displayed blinking and blocks other than the block 83 are displayed not highlighted (displayed blanked). By this, the user is guided that inhalation is finished and thus the user should move to exhalation.

At times t7 and t8 during a cool-down period, as with the warm-up period, fifth blocks 81 and 82 respectively located upward and downward from the center are fixedly displayed highlighted. In the breathing guide at time t7, the block 82 is displayed blinking and the first block located upward from the center is displayed highlighted. By this, the user is guided that an exhalation state should be continued for on the order of another one-half.

Note that the display mode of the breathing guide is not limited to highlight/no hyghlight such as that described above; for example, the display color of blocks may be changed.

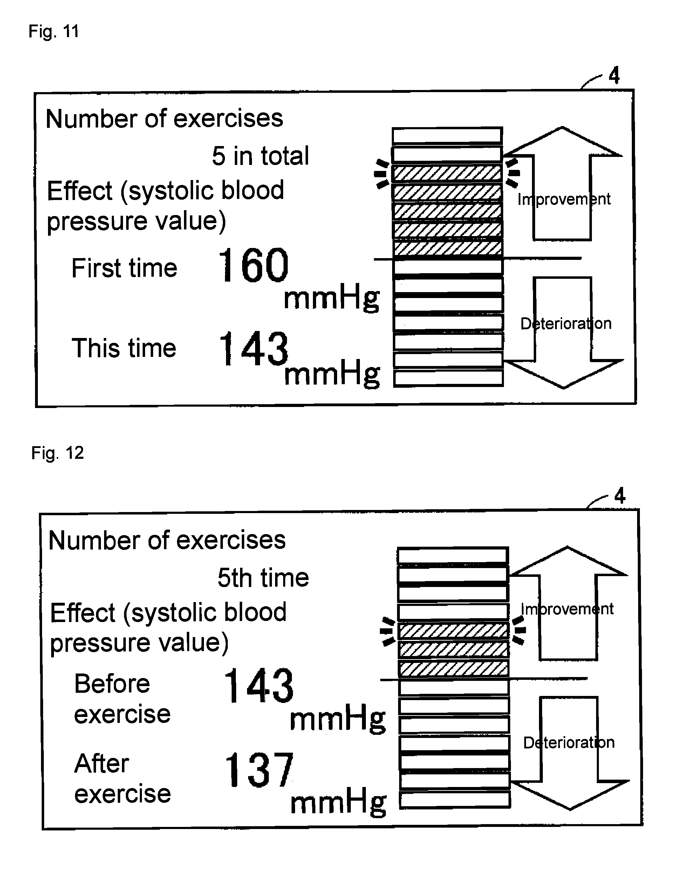

FIGS. 11 and 12 are diagrams each showing exemplary display of information on an effect index. FIG. 11 is a diagram showing an exemplary display of information on an effect index for before exercise in step S6 and FIG. 12 is a diagram showing an exemplary display of information on an effect index for before and after exercise in step S21. The exemplary display of information on an effect index for before exercise shown in FIG. 11 may be similar also for the case of an effect index for after exercise.

Referring to FIGS. 11 and 12, each effect index is, for example, level-displayed. Level-display is performed by highlighting/not highlighting 14 blocks, as with the breathing guide. Of the 14 blocks, seven blocks displayed on the upper side represent improvement and seven blocks displayed on the lower side represent deterioration. Note that it is assumed that levels (level -7 to level +7) are predetermined according to the value of the degree of decrease in blood pressure.

In FIG. 11, of the seven blocks located on the upper side, the first to fourth blocks from the center are displayed highlighted (displayed darkened) and the fifth block from the center is displayed blinking. By this, the user is informed that a blood pressure value for this time is reduced (improved) by five levels, as compared with a blood pressure value for the first time. When performing such level-display, it is desirable that, as shown in FIG. 11, the blood pressure value for the first time and the blood pressure value for this time are further displayed. By this, the user can grasp the degree of improvement/deterioration in greater detail.

In FIG. 12, of the seven blocks located on the upper side, the first and second blocks from the center are displayed highlighted (displayed darkened) and the third block from the center is displayed blinking. By this, the user is informed that a blood pressure value for after exercise is reduced (improved) by three levels, as compared with a blood pressure value for before exercise. In this case too, it is desirable that the blood pressure value (characteristic value) for before exercise and the blood pressure value (characteristic value) for after exercise are further displayed. By this, the user can grasp the degree of improvement/deterioration in characteristic value (blood pressure value) in greater detail.

Note that although here information on an effect index is informed by the level of improvement/deterioration, the present invention is not limited thereto; for example, the value of an effect index (the degree of decrease in blood pressure value) itself may be informed. When displaying information on an effect index, the number of exercises may be further displayed.

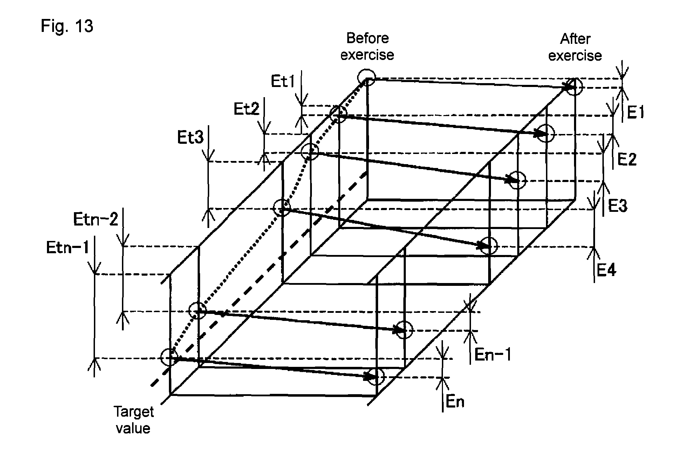

FIG. 13 is a diagram showing an exemplary display of the trend of the effect index in step S26. In FIG. 13, a trend of the effect index for before each exercise and a trend of the effect index for before and after exercise, for the first time to this time are displayed. In the drawing, E1, E2,..., En each represent an effect index for before and after exercise, i.e., an effect per exercise. Et1, Et2,..., Etn each represent an effect index for before each exercise, i.e., a cumulative effect of exercise according to the progression of exercise. By the trends of effect indices thus being displayed, the user can visually grasp short-term/long-term effects of exercise.

Note that instead of showing trends of effect indices for the first time to this time, trends of effect indices for a certain period of time (e.g., for one week) may be displayed.

An effect index for during exercise or for after each exercise may be further displayed. Alternatively, the user may be allowed to select an effect index he/she wants to display and a trend of the selected effect index may be displayed.

Note that although in the above description when the exercise index is a blood pressure value, the characteristic value calculating unit 203 calculates a blood pressure value itself as a characteristic value of the user, when the exercise index is other than the blood pressure value, specifically, a characteristic value such as that shown below is calculated.

When the exercise index is the above-described autonomic nervous activity level index, the characteristic value calculating unit 203 calculates, for example, a heart rate fluctuation as a characteristic value. When calculating a heart rate fluctuation, specific processes in steps S4, S14, and S20 in FIG. 4 are different from those for the case of a blood pressure value. First, the biological information detection control unit 202 performs detection control of biological information. Based on the detected biological information, the characteristic value calculating unit 203 calculates basic information, i.e., heart rate, to calculate a characteristic value. Thereafter, after a predetermined period of time has elapsed, the biological information detection control unit 202 performs detection control of biological information again. Based on the detected biological information, the characteristic value calculating unit 203 calculates basic information, i.e., heart rate, to calculate a characteristic value. Based on the two heart rates thus calculated, the characteristic value calculating unit 203 calculates a heart rate fluctuation (ΔHR). Note that for procedures of calculation of heart rate and a heart rate fluctuation any known procedure can be used. Note also that biological information for this case is information for calculating a heart rate fluctuation and thus may be electrocardiographic waveform information.

When calculating a heart rate fluctuation, in steps S4 and S20, it is desirable to inform the user at a time between the first biological information detection control and the second biological information detection control, to take a deep breath, for example, to display the message "Take a deep breath" on the display unit 4. By this, even before or after exercise, heart rates respectively for before and after a deep breathing state can be calculated. Note, however, that in view of providing stimulation to autonomic nerves, the user may be informed to take a quick breath. In step S14, even during breathing exercise, when performing the second biological information detection, it is desirable to inform to suspend deep breathing, for example, to display the message "Suspend breathing exercise" on the display unit 4. Note that a suspension period may be preset in an exercise pattern in advance.

In steps S6, S16, S21, and S22, the effect index calculating unit 204 calculates the degree of suppression of the autonomic nervous activity level based on two heart rate fluctuations (ΔHR).

When the exercise index is the above-described blood pressure stability index, the characteristic value calculating unit 203 calculates, for example, BRS (Baroreflex Sensitivity) as a characteristic value. The BRS is calculated by, for example, "blood pressure fluctuation (ΔBP)/heart rate fluctuation (ΔHR)". The BRS is an index indicating how much the blood pressure value increases when the heart rate is changed by providing stimulation to autonomic nerves by deep breathing. The higher the value, the more baroreflex sensitivity is reduced. That is, it can be evaluated that blood pressure stability is low and thus the user is unhealthy.

When calculating BRS too, in steps S4, S14, and S20 in FIG. 4, a process similar to that for when calculating a heart rate fluctuation is performed. Note that when calculating BRS, as the above-described basic information, a blood pressure value and heart rate are calculated, and based on the calculated two of blood value and heart rate, BRS is calculated.

In steps S6, S16, S21, and S22, the effect index calculating unit 204 calculates the degree of increase in blood pressure stability (ΔBRS) based on two BRSs.

Note that when heart rate and/or a blood pressure value can be continuously measured in synchronization with breathing, a heart rate fluctuation or BRS may be calculated by a single measurement operation (biological information detection process).

Although the above first embodiment describes that only one type of characteristic value and one type of effect index are calculated, two or more types of characteristic value and two or more types of effect index may be calculated and the two or more types of effect index may be informed to the user.

Alternatively, in the present embodiment, an effect index calculated based on two characteristic values is informed to the user. However, as long as the user can grasp an effect of breathing exercise, for example, instead of an effect index, two characteristic values (e.g., a characteristic value for before the first exercise and a characteristic value for before current exercise, etc.) may be informed to the user. In this case, the effect index calculating unit 204 in FIG. 2 may not be included in the control unit 20. Also, instead of step S6 in FIG. 4, a characteristic value for before current exercise calculated in step S4 and a characteristic value for before the first exercise are informed. Specifically, for example, these two characteristic values are simultaneously or alternately displayed on the display unit 4. Similarly, instead of step S16, a characteristic value for during exercise calculated in step S14 may be informed. Instead of step S21, a characteristic value for before current exercise calculated in step S14 and a characteristic value for after current exercise calculated in step S20 may be informed. Instead of step S22, a characteristic value for after current exercise calculated in step S20 and a characteristic value for after the first exercise may be informed. Instead of steps S24 and S26, a trend of a characteristic value may be informed. In the exercise pattern determination process shown in FIG. 5, in step S106, a stored value of the latest characteristic value (e.g., a characteristic value for before exercise) may be read, and an update of the pointer (S107) may be performed based on the latest characteristic value.

[Second Embodiment]
Next, a second embodiment of the present invention will be described.

Although in the first embodiment during breathing exercise an exercise pattern which is determined before the exercise is not changed, in the second embodiment even during breathing exercise the exercise pattern can be changed. Note that the basic configuration of a breathing exerciser in the second embodiment is similar to that of the breathing exerciser 100 in the first embodiment. Thus, also here description is made using the reference numerals used in the first embodiment.

Differences from the first embodiment will be described below.
Referring to FIG. 2, a control unit 20 of a breathing exerciser 100 in the second embodiment further includes a pattern changing unit 208, in addition to the units described in the first embodiment. The pattern changing unit 208 changes an exercise pattern being executed, based on an effect index for during a current exercise period.

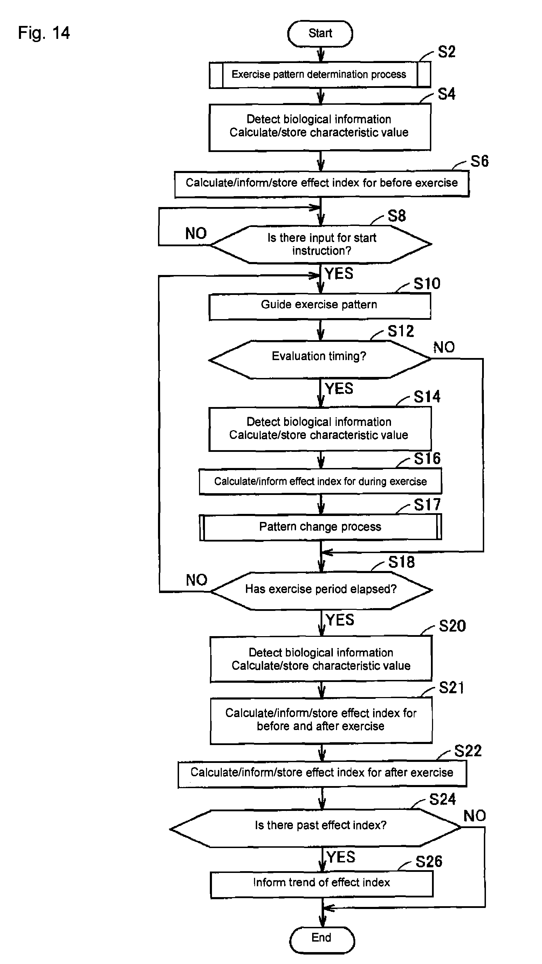

FIG. 14 is a flowchart showing a flow of a breathing exercise process in the second embodiment of the present invention. The process shown in the flowchart in FIG. 14 is stored in advance in a memory 12 as a program and a function of the breathing exercise process is implemented by the control unit 20 reading and executing this program. Note that processes similar to those shown in FIG. 4 are denoted by the same step numbers.

Referring to FIG. 14, in the breathing exercise process in the second embodiment, a pattern change process (step S17) is performed between steps S16 and S18.

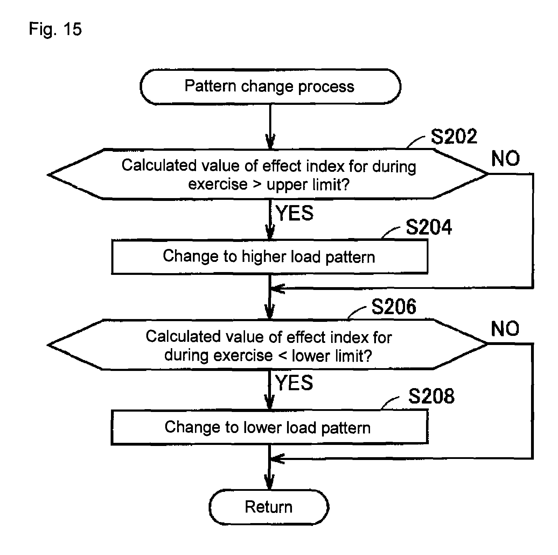

A flowchart of a subroutine showing the pattern change process is shown in FIG. 15. FIG. 15 is a diagram showing the pattern change process in the second embodiment of the present invention.

Referring to FIG. 15, first, the pattern changing unit 208 determines whether a calculated value of an effect index for during exercise exceeds a predetermined upper limit (step S202). If it is determined that the calculated value exceeds the upper limit (YES in step S202), then processing proceeds to step S204. On the other hand, if it is determined that the calculated value of an effect index for during exercise is less than or equal to the upper limit (NO in step S202), then processing proceeds to step S206.

In step S204, the pattern changing unit 208 changes an exercise pattern being executed to a higher load pattern. More specifically, for example, exercise time is made longer than a set value. Alternatively, the depth of breathing may be made greater than a set value. Alternatively, these may be combined or other parameters (e.g., the intensity of breathing, a ratio between an exhalation period and an inhalation period, etc.) may be changed. When the process in step S204 is completed, processing proceeds to step S206.

In step S206, the pattern changing unit 208 determines whether the calculated value of an effect index for during exercise falls below a predetermined lower limit. If it is determined that the calculated value falls below the lower limit (YES in step S206), then processing proceeds to step S208. On the other hand, if it is determined that the calculated value of an effect index for during exercise is less than or equal to the lower limit (NO in step S206), then processing is returned to the main routine.

In step S208, the pattern changing unit 208 changes an exercise pattern being executed to a lower load pattern. More specifically, for example, exercise time is made shorter than a set value. Alternatively, the depth of breathing may be made smaller than a set value. Alternatively, these may be combined or other parameters may be changed. When the process in step S208 is completed, processing is returned to the main routine.

As such, in the second embodiment, when such a real-time effect index as an effect index for during exercise exceeds a predetermined range, an exercise pattern can be flexibly changed to a pattern appropriate for the user.

Note that the upper and lower limits used in the pattern change process may be the same as those used when determining an exercise pattern in the first embodiment.

Although in the second embodiment an exercise pattern is changed based on an effect index for during exercise, an exercise pattern may be changed based on a characteristic value for during exercise. In this case too, for example, an exercise pattern is changed according to whether a characteristic value for during exercise exceeds a predetermined upper limit or exceeds a predetermined lower limit.

A breathing exercise method to be performed by the breathing exerciser of the present invention can also be provided in the form of a program. Such a program can also be provided in the form of a program product by storing the program on an optical medium, such as a CD-ROM (Compact Disk-ROM), or in a computer-readable storage medium, such as a memory card. Alternatively, the program can also be provided by download via a network.

A program product to be provided is executed by being installed in a program storage unit such as the memory 12. Note that the program product includes a program itself and a storage medium storing the program.

The embodiments disclosed herein are to be considered in all respects as illustrative and not restrictive. The scope of the present invention is indicated by the appended claims rather than by the foregoing description, and all changes that come within the meaning and range of equivalency of the appended claims are intended to be embraced therein.

## Claims

1. A breathing exerciser comprising:
a guide unit (201) for guiding an exercise pattern of breathing to a user;
a detecting unit (30) for detecting biological information on the user;
a detection control unit (202) for controlling detection of the biological information at timing associated with an exercise period during which the exercise pattern is guided;
a characteristic value calculating unit (203) for calculating a characteristic value that reflects an exercise index, based on the biological information detected according to the control of the detection control unit, the exercise index being a target of breathing exercise;
an effect index calculating unit (204) for calculating an effect index based on at least two characteristic values, the effect index representing an effect of the breathing exercise; and
an informing unit (206) for informing the effect index to the user.

2. The breathing exerciser according to claim 1, wherein
the detection control unit allows the detecting unit to detect biological information before and after the exercise period,
the characteristic value calculating unit calculates the two characteristic values based on the detected biological information respectively for before and after the exercise period, and
the effect index calculating unit calculates an effect index for before and after the exercise period, based on the calculated characteristic values.

3. The breathing exerciser according to claim 1 further comprising: a storage unit (12) for storing a characteristic value which is based on the biological information detected before or after the previous exercise period, wherein
the detection control unit allows the detecting unit to detect the biological information before or after the exercise period,
the characteristic value calculating unit calculates the two characteristic values based on the detected biological information and the biological information stored in the storage unit, and
the effect index calculating unit calculates the effect index for before each exercise period or for after each exercise period, based on the calculated characteristic values.

4. The breathing exerciser according to claim 1, wherein
the detection control unit allows the detecting unit to detect the biological information during the exercise period,
the characteristic value calculating unit calculates the characteristic value based on the detected biological information for during the exercise period, and
the effect index calculating unit calculates the effect index for during the exercise period, based on the calculated characteristic value.

5. The breathing exerciser according to claim 4 further comprising: a changing unit (208) for changing the exercise pattern based on a result of comparison between the effect index for during the exercise period which is calculated by the effect index calculating unit and a predetermined threshold.

6. The breathing exerciser according to claim 1, wherein
the exercise index and the characteristic value each are a blood pressure value, and
the effect index is a degree of decrease in the blood pressure value.

7. The breathing exerciser according to claim 1, wherein
the exercise index is an autonomic nervous activity level index representing an autonomic nervous activity level,
the characteristic value includes a heart rate fluctuation,
the characteristic value calculating unit calculates heart rate fluctuations based on two heart rates respectively corresponding to two pieces of detected biological information, and
the effect index calculating unit calculates, as the effect index, a degree of suppression of the autonomic nervous activity level based on the two heart rate fluctuations.

8. The breathing exerciser according to claim 1, wherein
the exercise index is a blood pressure stability index,
the characteristic value includes a baroreflex sensitivity,
the characteristic value calculating unit calculates the baroreflex sensitivities based on a heart rate fluctuation and a blood pressure fluctuation respectively corresponding to two pieces of detected biological information, and
the effect index calculating unit calculates, as the effect index, a degree of increase in blood pressure stability based on a difference or ratio between the two baroreflex sensitivities.

9. The breathing exerciser according to claim 1 further comprising: a storage unit (12) for storing the effect index associated with the previous exercise period, wherein
the informing unit further informs a trend between the stored effect index and the calculated effect index.

10. The breathing exerciser according to claim 1 further comprising:
an input unit (21) for inputting physical information on the user; and
a determining unit (20) for determining an exercise pattern based on the inputted physical information, wherein
the guide unit guides the determined exercise pattern.

11. The breathing exerciser according to claim 10, wherein the determining unit determines the exercise pattern based on the effect index associated with the previous exercise period.

12. A breathing exerciser comprising:
a guide unit (201) for guiding an exercise pattern of breathing to a user;
a detecting unit (30) for detecting biological information on the user;
a detection control unit (202) for controlling detection of the biological information at timing associated with an exercise period during which the exercise pattern is guided;
a characteristic value calculating unit (203) for calculating a characteristic value that reflects an exercise index, based on the biological information detected according to the control of the detection control unit, the exercise index being a target of breathing exercise; and
an informing unit (206) for informing at least two characteristic values to the user.

13. The breathing exerciser according to claim 12, wherein
the detection control unit allows the detecting unit to detect the biological information before and after the exercise period,
the characteristic value calculating unit calculates the two characteristic values based on the detected biological information respectively for before and after the exercise period, and
the informing unit informs the characteristic values respectively for before and after the exercise period.

14. The breathing exerciser according to claim 12 further comprising: a storage unit (12) for storing a characteristic value which is based on the biological information detected before or after the previous exercise period, wherein
the detection control unit allows the detecting unit to detect the biological information before or after the exercise period,
the characteristic value calculating unit calculates the two characteristic values based on the detected biological information and the biological information stored in the storage unit, and
the informing unit informs the characteristic values for before each exercise period or for after each exercise period.

15. The breathing exerciser according to claim 12, wherein
the detection control unit allows the detecting unit to detect the biological information during the exercise period,
the characteristic value calculating unit calculates the characteristic value based on the detected biological information for during the exercise period, and
the informing unit informs the characteristic value for during the exercise period.

16. The breathing exerciser according to claim 15 further comprising: a changing unit (208) for changing the exercise pattern based on a result of comparison between the characteristic value for during the exercise period which is calculated by the characteristic value calculating unit and a predetermined threshold.

17. The breathing exerciser according to claim 12, wherein
the exercise index and the characteristic value each are a blood pressure value.

18. The breathing exerciser according to claim 12, wherein
the exercise index is an autonomic nervous activity level index representing an autonomic nervous activity level,
the characteristic value includes a heart rate fluctuation, and
the characteristic value calculating unit calculates heart rate fluctuations based on two heart rates respectively corresponding to two pieces of the detected biological information.

19. The breathing exerciser according to claim 12, wherein
the exercise index is a blood pressure stability index,
the characteristic value includes a baroreflex sensitivity, and
the characteristic value calculating unit calculates the baroreflex sensitivities based on a heart rate fluctuation and a blood pressure fluctuation respectively corresponding to two pieces of detected biological information.

20. The breathing exerciser according to claim 12 further comprising:
an input unit (21) for inputting physical information on the user; and
a determining unit (20) for determining an exercise pattern based on the inputted physical information, wherein
the guide unit guides the determined exercise pattern.

21. The breathing exerciser according to claim 20, wherein the determining unit determines the exercise pattern based on the effect index associated with the previous exercise period.
